# EUROPEAN PATENT APPLICATION

(11) **EP 4 574 032 A1**
(43) Date of publication of application: **25.06.2025**
(21) Application number: 24221998.8
(22) Date of filing: 20.12.2024
(51) Int. Cl.: A61B 5/0538, A61B 5/06, A61B 5/287, A61B 5/367, A61B 5/00

(54) **COMPUTER METHOD, SYSTEM, AND GUI FOR REAL-TIME VISUAL FEEDBACK OF INTRALUMINAL CATHETER ENGAGEMENT**

(30) Priority: 22.12.2023 US 202318393767
(71) Applicant: Biosense Webster (Israel) Ltd., 2066717 Yokneam (IL)
(72) Inventor: GLINER, Vadim, 2066717 Yokneam (IL); AVNI, Uri, 2066717 Yokneam (IL); LAMHOT, Yoav, 2066717 Yokneam (IL)
(74) Representative: Carpmaels & Ransford LLP

(57) **Abstract**

The presently disclosed subject matter includes a computer system, method, and graphical user interface that provide graphical feedback indicating real-time proximity based on changes in electrical impedance sensed by electrodes of a catheter. As the impedance is related to proximity of the electrodes to the tissue walls, the system utilizes impedance measurements to visually alter appearance of a graphical representation of the electrodes according to changes in the sensed impedance. The graphical feedback enables the physician to make real-time adjustments to the catheter's positioning and applied force, and thereby enhance the precision and effectiveness of Intraluminal catheter therapy.

## Description

### FIELD OF THE PRESENTLY DISCLOSED SUBJECT MATTER

The presently disclosed subject matter relates to intraluminal catheter therapy for the diagnosis and treatment of medical disorders.

### BACKGROUND

Intraluminal catheter therapy (ICT) or catheterization has become a pivotal instrument in the field of medicine for both the diagnosis and treatment of various medical disorders. This minimally invasive procedure involves guiding a slender, flexible tube, or catheter, into a luminal organ. Equipped with electrodes, the catheter is used, *inter alia,* for mapping the lumen, and identifying precise locations related to abnormal medical conditions.

Cardiac ICT is an important tool for both the diagnosis and treatment of cardiac disorders, particularly arrhythmias. This involves inserting a catheter equipped with electrodes through the blood vessels and into the heart, using the catheter for generating a map of the heart's electrical activity, and identifying the precise locations of abnormal electrical activity. The identified sites can then be treated through ablation, where targeted energy neutralizes the abnormal tissue, restoring normal heart rhythm. This integrated approach has revolutionized cardiac care, offering patients less invasive options with shorter recovery times.

### BRIEF DESCRIPTION OF THE DRAWINGS

In order to understand the presently disclosed subject matter and to see how it may be carried out in practice, the subject matter will now be described, by way of non-limiting examples only, with reference to the accompanying drawings, in which:
**Fig. 1** is a schematic pictorial illustration of an example of a catheter-based electrophysiology mapping and ablation system;
**Fig. 2** is a more detailed isometric view of an expandable end assembly on the distal tip of the catheter of Figure 1;
**Fig. 3a** is a block diagram schematically illustrating a processing circuitry configured to provide graphical feedback indicating real-time proximity, in accordance with some examples of the presently disclosed subject matter;
**Fig. 3b** is a block diagram schematically illustrating a graphical proximity feedback engine implemented as part of the processing circuitry shown in Fig. 3a, in accordance with some examples of the presently disclosed subject matter;
**Fig. 4** is a simplified flow chart of an example method for providing graphical feedback on real-time proximity of electrodes on a catheter to a cavity wall, in accordance with an example of the presently disclosed subject matter; and
**Fig. 5** is a graph corresponding to an impedance-proximity profile, in accordance with some examples of the presently disclosed subject matter.

### OVERVIEW

An important aspect of catheterization in the context of cardiology is related to tissue contact and ablation accuracy. Ensuring adequate contact between the catheter distal end assembly (e.g., balloon and/or basket) located at its distal end and the heart tissue is crucial for accurate and effective results.

A physician manipulating a catheter would benefit from knowing how much force is being applied on the tissue wall. The force distribution on the distal end assembly can help the physician to accurately steer the end. This information can be used for example during mapping of the internal surface of a luminal organ such as the heart. During mapping, multiple points are collected by the catheter from the surface of the internal cavity wall. To obtain accurate mapping, it is desired to avoid the application of excessive force on the tissue when manipulating the catheter, as this may result in deformation of the tissue surface, e.g., tenting, and consequently cause inaccurate mapping output. An indication of force being applied on the tissue walls is also helpful during various other procedures such as Pulsed Field Ablation (PFA). Too little contact can render the ablation ineffective, while too much pressure can cause excessive tissue damage. An indication of the proximity between the catheter and tissue can help the physician reach a threshold level of force to achieve a desired lesion depth.

The presently disclosed subject matter includes a computer system, method, and graphical user interface that provide graphical feedback indicating real-time proximity based on changes in electrical impedance sensed by electrodes of a catheter. As the impedance is related to proximity of the electrodes to the tissue walls, the system utilizes impedance measurements to visually alter appearance of a graphical representation of the electrodes according to changes in the sensed impedance. The graphical feedback enables the physician to make real-time adjustments to the catheter's positioning and applied force, and thereby enhance the precision and effectiveness of ICT. For instance, if the impedance suggests inadequate contact, the physician may reposition the catheter to achieve better engagement with the tissue. Conversely, a high impedance reading could signal excessive pressure, prompting the physician to reduce the contact force to prevent tenting and/or potential tissue damage.

### DETAILED DESCRIPTION

In the drawings and descriptions set forth, identical reference numerals indicate those components that are common to different embodiments or configurations. Elements in the drawings are not necessarily drawn to scale.

Unless specifically stated otherwise, as apparent from the following discussions, it is appreciated that, throughout the specification, discussions utilizing terms such as "displaying", "determining", "updating", "generating" or the like, include an action and/or processes of a computer that manipulate and/or transform data into other data, said data represented as physical quantities, e.g. such as electronic quantities, and/or said data representing the physical objects.

The terms "computer", "computer system", "computer device", or the like, should be expansively construed to include any kind of hardware-based electronic device with one or more data processing circuitries. Each processing circuitry can comprise, for example, one or more processors operatively connected to (including non-transitory) computer memory, loaded with executable instructions for executing operations, as further described below .

The one or more processors referred to herein can represent for example, one or more general-purpose processing devices such as a microprocessor, a central processing unit, or the like. More particularly, a given processor may be one of: a complex instruction set computing (CISC) microprocessor, a reduced instruction set computing (RISC) microprocessor, a very long instruction word (VLIW) microprocessor, processor implementing other instruction sets, or a processor implementing a combination of instruction sets. The one or more processors may also be one or more special-purpose processing devices such as an application specific integrated circuit (ASIC), a field programmable gate array (FPGA), a digital signal processor (DSP), a graphics processing unit (GPU), a network processor, or the like.

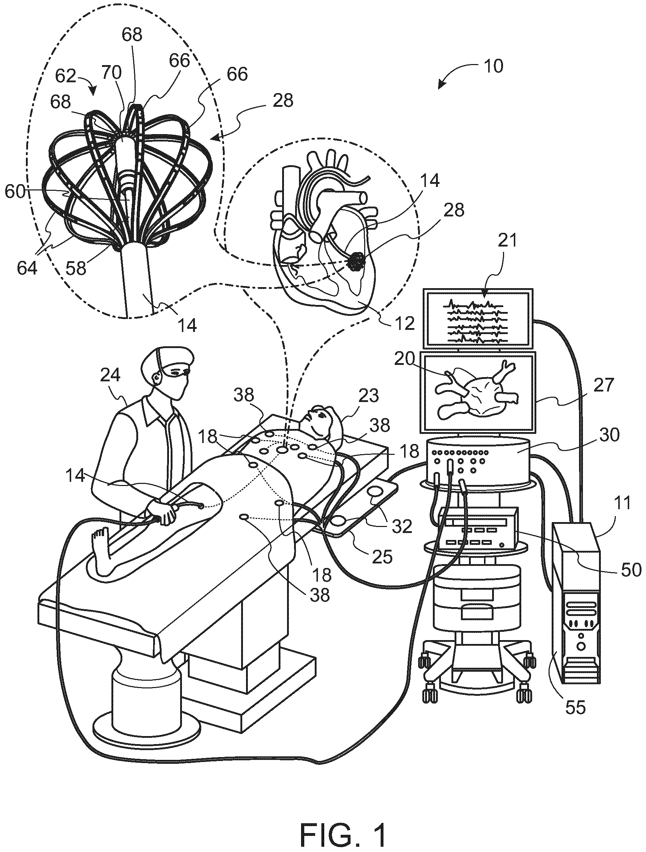

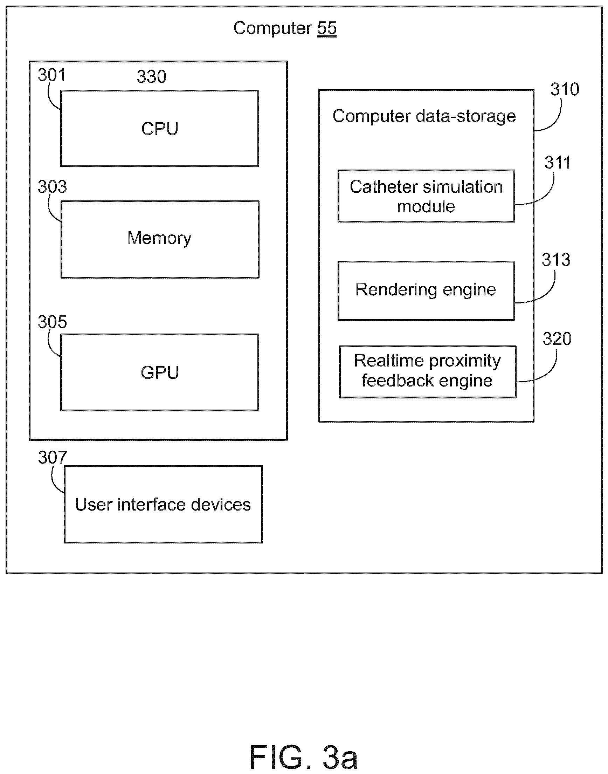

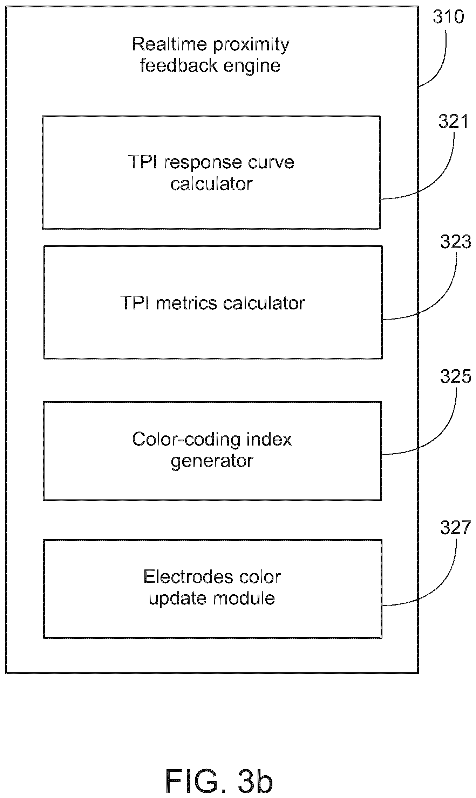

Figs. 1, 3a, and 3b illustrate schematics of the system architecture in accordance with certain examples of the presently disclosed subject matter. Elements in Figs. 1, 3a, 3b, and 2 can be made up of any combination of software and hardware and/or firmware that performs the functions as defined and explained herein. Elements in Figs. 1, 3a, and 3b may be centralized in one location or dispersed over more than one location. In some examples, certain operations can be implemented by a remote cloud computing infrastructure, where information is transmitted from computer 55 to the cloud, where processing is executed and the processing output is sent back to computer 55.

The term luminal organ refers to any organ that has a lumen, i.e., an interior space, cavity, or channel. Luminal organs include, for example, blood vessels, kidney, bladder, urethra, heart, and colon.

Bearing the above in mind, attention is drawn to Fig. 1 showing an example of an ICT system. More specifically, Fig. 1 shows a catheter-based electrophysiology mapping (also referred to herein as "cardiac mapping") system 10. In some cases, system 10 can be also used for ablation. System 10 includes one or more catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, one or more catheters may be inserted into the delivery sheath catheter to arrive at the desired location in heart 12. Catheter types may include, for example, catheters dedicated for sensing Intracardiac Electrogram (IEGM) signals, catheters dedicated for ablating, and/or catheters dedicated for both sensing and ablating. An example catheter 14 is illustrated herein. In some examples physician 24 places a distal end assembly 28 of catheter 14 in contact with the heart wall for sensing a target site in heart 12. For ablation, physician 24 may similarly place a distal end of an ablation catheter in contact with a target site for ablating tissue.

Catheter 14 is a non-limiting example of a catheter that includes one and preferably multiple electrodes 66 distributed over distal end assembly, e.g., comprising a plurality of splines 62 at distal end assembly 28. In addition to electrodes, catheter 14 may additionally include a position sensor 29 embedded in or near distal tip 28 for tracking position and orientation of distal end assembly 28. In some examples, position sensor 29 is a magnetic based position sensor including three magnetic coils for sensing three-dimensional (3D) position and orientation.

Electrical activity at points within the heart is typically sensed and measured by advancing a catheter containing one or more electrical sensors into a chamber of the heart and acquiring data at a multiplicity of points. These data are then utilized to compute an electro-anatomical map of the heart chamber or a portion thereof.

Magnetic based position sensor 29 may be operated together with a location pad 25 including a plurality of magnetic coils 32 configured to generate magnetic fields in a predefined working volume. Real time position of distal end of a shaft of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 may further include one or more electrode patches 38 positioned for skin contact on patient 23 to establish location reference for location pad 25 as well as impedance-based tracking of electrodes 26. For impedance-based tracking, electrical current is directed to electrodes 26 and sensed at electrode skin patches 38 so that the location of each electrode can be triangulated via the electrode patches 38. Details of impedance-based location tracking technology are described in US Patent Nos. 7,536,218; 7,756,576; 7,848,787; 7,869,865; and 8,456,182.

Electrodes 66 may also be configured for receiving an AC signal while being paired with a reference electrode. Impedance in response to the AC signal may be sensed and used to determine local proximity between an electrode 66 and the tissue wall, e.g., cavity wall. An example method for assessing proximity based on impedance is described for example in US Patent No. 20210177504.

A recorder 11 records and displays electrograms 21 captured with body surface ECG electrodes 18 and intracardiac electrograms (IEGMs) captured with electrodes 66 of catheter 14. Recorder 11 may include pacing capability for pacing the heart rhythm and/or may be electrically connected to a standalone pacer.

In some examples, system 10 includes an ablation energy generator 50 that is adapted to conduct ablative energy to one or more of the electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

In some examples, system 10 further includes patient interface unit (PIU) 30, which is an interface device configured to establish electrical communication between catheters, other electrophysiological equipment, power supply, and a workstation 55 for controlling operation of system 10. Electrophysiological equipment of system 10 may include, for example, multiple catheters, location pad 25, body surface ECG electrodes 18, electrode patches 38, ablation energy generator 50, and recorder 11. According to some examples, PIU 30 additionally includes processing capability for implementing real-time computations of location of the catheters and for performing ECG calculations.

Workstation 55 includes a processing circuitry comprising one or more processors operatively connected to a computer memory of some sort. An appropriate operating software and user interface capability can be executed by the processing circuitry. Workstation 55 may provide multiple functions, optionally including, for example:
- Modeling the endocardial anatomy in three-dimensions (3D) and rendering a 3D graphical representation of the model or anatomical map 20 for display on a display device 27.
- Displaying on display device 27 activation sequences (or other data) compiled from recorded electrograms 21 in representative visual indicia (e.g., by color coding) or imagery superimposed or overlaid on the rendered anatomical map 20.
- Rendering a real-time 3D graphical representation of the distal end assembly 28 for display on a display device 27.
- Displaying real-time location and orientation of one or more catheters within the heart chamber.
- Displaying on display device 27 sites of interest such as places where ablation energy has been applied.

One commercial product embodying elements of the system 10 is available as the CARTO^{™} 3 System, available from Biosense Webster, Inc., 31A Technology Drive, Irvine, CA 92618.

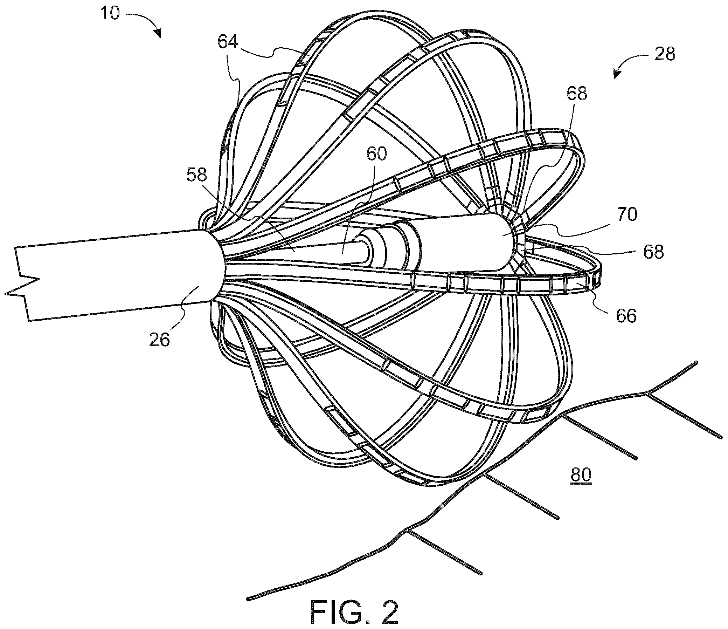

Reference is now made to Figure 2, which is a more detailed isometric view of an expandable end assembly on the distal tip of the catheter of Figure 1.

The distal end assembly 28 is an expandable assembly formed with a plurality of splines 64. In some examples, each of splines 64 includes a plurality of electrodes, e.g., 2-10 electrodes 66 configured for sensing IEGM.

Fig. 3a is a block diagram schematically illustrating a computer comprising a processing circuitry configured for executing data processing operations related to graphical feedback indicating real-time proximity, according to certain examples of the presently disclosed subject matter. Computer 55 (otherwise referred to hereinabove as "workstation 55") is operatively connected to system 10 disclosed in Fig. 1. Notably, in some examples certain operations described with reference to computer 55 are performed by PIU 30.

Computer 55 comprises a processing circuitry 330 comprising at least one computer processor operatively connected to a computer memory 303. For example, a central processing unit (CPU 301) can be configured to perform various calculations and data processing required for generating and rendering 3D graphics of the catheter while being inserted into the heart. The memory is configured for storing the relevant software, including for example computer instructions dedicated for generating and updating the 3D graphics of the heart. Processing circuitry can further comprise a graphics processing unit (GPU) 305 configured to expedite graphics rendering tasks, including drawing shapes and applying textures swiftly and concurrently. Data storage device 310 is configured to store various data such as computer programs, files, textures, and software tools including user interface software. Software and other data stored on data storage device 310 can be uploaded to memory 303 during execution. As explained above with reference to Fig. 1, computer 55 is operatively connected to a display device (e.g., display 27 shown in Fig. 1), as well as other user interacting devices 307 such as computer mouse and keyboard, to enable viewing and interacting with the 3D graphics and other software and hardware tools.

As will be further detailed with reference to the figures below, processing circuitry 330 can be configured to execute several functional modules in accordance with computer-readable instructions implemented on a non-transitory computer-readable storage medium. By way of example, such functional modules are illustrated within data-storage device 310.

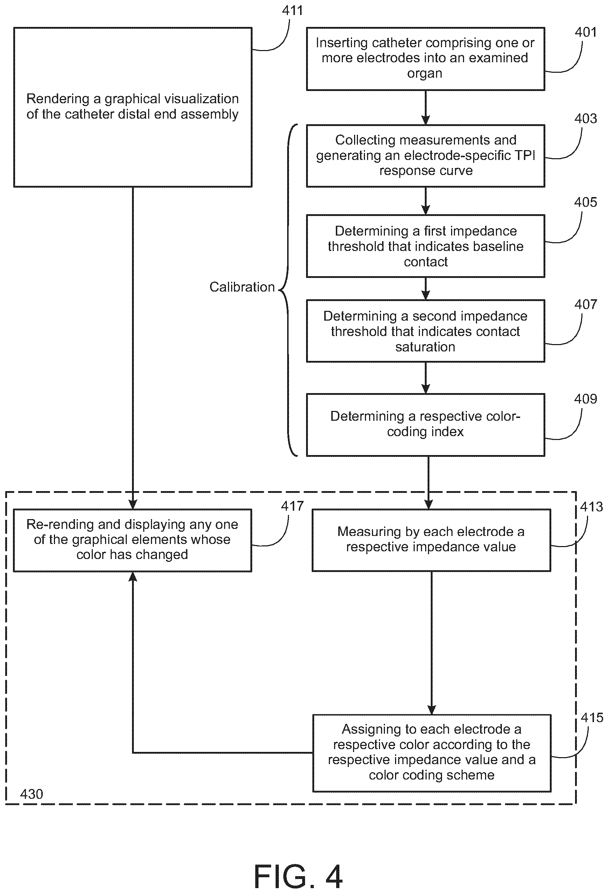

Fig. 3a shows by way of example, catheter simulation module 311 configured in general to generate a graphical simulation of the catheter distal end, rendering engine 313 configured to render various graphics including the graphical representation of the catheter distal end, and real-time proximity feedback engine 320 configured to generate and provide real-time proximity feedback as further described below. An example of a more detailed description of components of real-time proximity feedback engine 320 is shown in Fig. 3b and described with reference to operations in Fig. 4.

Fig. 4 is a flowchart of operations carried out as part of a cardiac mapping procedure, according to certain examples of the presently disclosed subject matter. For the sake of clarity and by way of non-limiting example, description of operations in Fig. 4 is made with reference to components shown in Fig. 1, Fig. 3a, and 3b.

As explained above, during Intraluminal Catheter Therapy (ICT), a catheter carrying multiple electrodes is inserted into a patient's luminal organ (401). Proximity detection of the catheter to the tissue walls of the organ is imperative for the completion of various tasks including the mapping of the internal surface of the organ and the accurate application of medical procedures within the organ, such as ablations performed within the heart chambers.

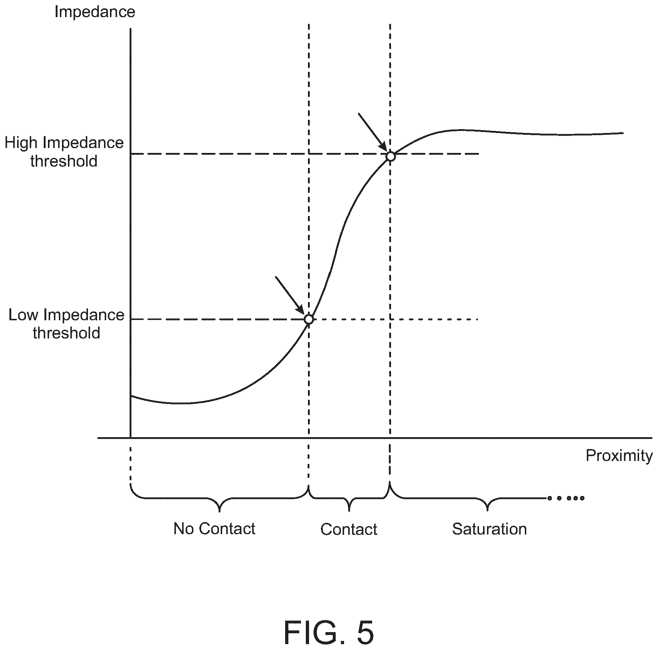

Tissue proximity index (TPI) represents the relation between the impedance measured by an electrode and its proximity to a tissue wall. Fig. 5 is a graph depicting a TPI profile (impedance-proximity profile), where the x-axis corresponds to the proximity (inverse of distance) of the catheter from the tissue, and the y-axis corresponds to impedance values.

Changes in impedance result from the difference between the electrical properties of the tissue and other mediums such as air or blood that the electrode is in contact with when not in contact with the tissue walls. In cardiac ICT, the catheter is typically first inserted into a major blood vessel and then navigated into the heart. Initially, the catheter is in contact with the blood, which has a lower impedance. The catheter is then carefully advanced towards the heart tissue until it makes contact with the tissue walls. The tissue walls are characterized by a higher impedance compared to blood, which can be detected and used to confirm proper contact between the catheter and the heart tissue.

As shown in Fig. 5, the graph is non-linear and can be divided into three distinct phases, including a non-contact phase, a contact phase, and a contact saturation phase. In the initial non-contact phase, the electrode is in contact with the blood and does not touch the tissue walls. At this phase the measured impedance is relatively low. As the electrode is moved closer to the tissue wall the measured impedance increases. At a certain point that marks the initial contact of the electrode with the tissue, a sharp increase in the measured impedance is observed. After this steep rise, the graph levels off into a plateau and enters a saturation phase. During this phase, the electrode is in full contact with the tissue and the application of additional pressure to the electrode does not result in a significant, if any, increase in impedance measurements.

While the general TPI profile shown in Fig. 5 is known, each electrode is associated with a different TPI (or impedance-proximity) response curve, depending on the particular impedance values it measures. The difference in values can result for example from the distance of each electrode from a reference electrode, e.g., fixed to the catheter and used for measuring impedance values.

Therefore, to facilitate proximity detection based on the electrodes' impedance measurements, a calibration procedure is implemented as the catheter is initially inserted into the tested organ. Operations listed as part of the calibration procedure are performed for each electrode on the catheter. As part of the calibration procedure, a respective TPI response curve is determined for each electrode located on the distal end assembly (block 403; e.g., by TPI response-curve calculator 321 in real-time proximity feedback engine 310). The response curve defines the relation between impedance measurements and proximity as observed by a particular electrode.

Considering one electrode on the catheter, following insertion of the catheter into the heart, for a short period (e.g., two seconds or less) a collection of impedance measurement values, obtained by the electrode while the catheter is being manipulated by the physician, are recorded and processed. A minimum impedance measurement value and a maximum impedance measurement value are extracted from the collection of impedance measurement values. The minimal impedance value is set as the X=0 value in the graph, and the maximal impedance value is set as the maximal impedance value preceding the plateau. The y-axis, representing the measured impedance values, is scaled, so its maximum and minimum align with the maximum and minimum values.

Notably, when calibrating multiple electrodes on a catheter, redundant electrodes can be used for accelerating the process of calculating a response curve for each electrode. Knowing the distance of each electrode from a fixed reference point (e.g., reference electrode) allows to adjust for any distance-related effects systematically. This facilitates the application of consistent corrections to all electrodes, adapted according to their individual distances, which effectively streamlines the calibration process. Adjustments to one electrode inform the required adjustments for others, making the process more efficient and ensuring consistency across the catheter.

According to the presently disclosed subject matter, once a scaled TPI response curve has been obtained for a given electrode, two threshold points are determined for the electrode (blocks 405 and 407; e.g., by TPI metric calculator 323 in real-time proximity feedback engine 310). The first threshold indicates an impedance value measured at a baseline or initial contact with the tissue (marked by the left arrow in Fig. 5), and the second threshold indicates an impedance value measured before the plateau of contact saturation (marked by the right arrow in Fig. 5).

According to one example, the first threshold can be determined based on some predefined low percentile from the minimum, and the second threshold can be determined based on some predefined high percentile from the maximum. For example, the first threshold can be set according to the 50^{th} percentile, namely defining impedance measurements above this percentile to indicate contact. The second threshold can be set according to the 90^{th} percentile, namely defining impedance measurements above this percentile to indicate contact saturation.

Following the determination of the first threshold and second threshold, a respective graphical features index is determined for each electrode (block 409; e.g., by color-coding index generator 325 in real-time proximity feedback engine 310). The graphical features index is used for translating impedance values into respective graphical elements that visually represent the different values, and provide, to a medical practitioner, real-time graphical feedback regarding the proximity of an electrode to a tissue wall of the luminal organ. The feedback provides the medical practitioner with information regarding the quality or degree of engagement (contact) between the catheter distal end and the tissue wall.

According to one example, the graphical features are colors, and the graphical feature index is implemented as a color-coding index, which is used for translating impedance values into respective colors used for coloring a respective graphical element representing the electrode in the 3D graphical visualization of the catheter. Notably, the term color-coding is used to include any type of color model, including but not limited to, grey scale model, RGB (Red, Green, Blue) model, HSV (Hue, Saturation, Value) model, etc. Other possible types of graphical features include using different shapes, where different shapes are used for indicating different impedance values, and varying flashing frequencies, where different flashing frequencies are used for indicating different impedance values. It is noted that, while the following description is made in relation to a color-coding index, this is done by way of example only, and other types of visual features are also contemplated to be within the scope of the presently disclosed subject matter.

The color-coding index associates a first color with impedance values below the first threshold, a second color with impedance values above the second threshold, and a plurality of additional colors with a plurality of respective impedance values (or range of values) between the first threshold value and the second threshold value.

Since each electrode may have a different TPI response curve, the respective color-coding index may also be different, namely different impedance values are associated with different colors. Thus, in some examples, for each electrode color-coding scale is applied on the range of values between the first threshold and the second threshold.

Assuming, for example, greyscale colors are used, impedance values below the first threshold are represented by black color, impedance values above the second threshold are represented by white color, and the range of impedance values between the first threshold and second threshold can be represented by a plurality of other shades of grey in a color-coding scale evenly distributed within the range. Considering a non-limiting example, where the range of impedance values is divided by a color-coding scale of evenly distributed three colors, the impedance values within the range can be divided into three, and associated with the respective grayscale values, 63, 127, and 191, with 0 representing the first threshold and 255 representing the second threshold. If the range of impedance values are divided by a color-coding scale of evenly distributed five colors, the impedance values within the range can be divided into five and associated with the respective grayscale values, 42, 85, 127, 170, and 212.

A graphical visualization of the catheter with multiple electrodes placed thereon, which graphically represents the catheter, is generated (block 411). This can occur, for example, before or during the calibration procedure.

In some examples, a three-dimensional (3D) representation graphically visualizing the catheter is generated with the help of catheter simulation module 311 and rendering engine 313 operating in association with CPU 301 and GPU 305. Catheter simulation module 311 is configured to generate graphics that replicate the catheter's geometry, volumes, surface topology, features, textures, etc. The graphics depict the catheter distal end assembly (e.g., catheter basket) and graphical elements which represent the electrodes and optionally also other components, in a manner that clearly shows their placement and size. Rendering engine 313 is configured to render the graphics for display on a display device 27. In some examples, graphical representation of the tissue walls surrounding the catheter is also provided.

Electrodes color update module 327 in real-time proximity feedback module 310, is configured to continuously receive impedance measurements values from each electrode and provide a respective color suitable for coloring the electrode according to the impedance values measured in real-time. The coloring of each electrode can be provided to GPU 305 and used by it during rendering the 3D graphics. During initial generation of the 3D graphics, default values can be assigned to all electrodes, e.g., coloring all electrodes in black.

Following the calibration phase, the execution phase is initiated (block 430). During this phase, while the catheter is being manipulated within the luminal organ (e.g., heart), each electrode continuously measures impedance values (block 413). In response to a change in the impedance value measured by the electrode, the color-coding index is applied for selecting a matching color according to the updated impedance value (block 415).

Each time an impedance measurement update is received, the graphical element representing the respective electrode that measured the value is re-rendered using the updated color selected according to the recently measured impedance value (block 417).

In some examples, electrode color update module 327 receives a stream of impedance measurements, uses the color-coding index, stored for example, in computer memory 303, for determining the matching color, and provides the color to the rendering engine 313, which re-renders the respective graphical element using the matching color. The GPU can be configured to execute shading programs configured to color the different graphical elements according to the data received from electrode color update module 327. The updated 3D visual representation of the catheter is displayed on the display device 27.

The operations associated with block 430 are continuously performed as the catheter is manipulated within the luminal organ. This ensures that the 3D graphics are dynamically updated to reflect the varying proximity of the electrodes to the tissue walls. Consequently, this provides continuous, real-time color-feedback to the medical partitioner that indicates the quality of the engagement of the catheter with the tissue walls.

During a medical procedure, such as mapping of the internal surface of the luminal organ, or an ablation as the catheter is being manipulated by a physician within the organ, the physician can view the 3D catheter graphics displayed on the screen and use the color-feedback of the catheter graphics generated in real-time to bring the catheter into contact with the tissue and adjust the degree of contact, and manage the force applied on the tissue to a desired level, thereby enhancing the accuracy and efficiency of the medical procedure.

According to a first aspect of the presently disclosed subject matter there is provided a computer-implemented method of providing real-time visual feedback of intraluminal catheter engagement using a catheter that comprises one or more electrodes placed on a catheter distal end assembly, the method comprising:
while a catheter is in a luminal organ of a patient:
rendering on a display a graphical representation of the catheter distal end assembly and the one or more electrodes thereon;
for each electrode of the one or more electrodes:
   identifying a range of impedance between a first threshold value corresponding to baseline tissue contact and a second threshold value corresponding to contact saturation for each of the one or more electrodes;
   defining a graphical feature index that associates impedance values within the defined range with respective visual features of the graphical representation of the one or more electrodes;
   repeatedly measuring impedance at each of the one or more electrodes; and
   dynamically updating a respective visual feature of the graphical representation of each of the one or more electrodes based on the impedance measured and the graphical feature index, thereby providing dynamic visual feedback indicative of quality of intraluminal catheter engagement with a tissue wall.

In addition to the above features, the method according to this aspect of the presently disclosed subject matter can optionally comprise one or more of features (i) to (vii) below, in any desired and technically possible combination or permutation:
i. Wherein the luminal organ is the heart of the patient.
ii. Wherein the distal end assembly is a basket comprising multiple splines, the electrodes being distributed on the splines.
iii. The method further comprising:
   obtaining by the electrode a plurality of impedance values;
   identifying a maximal impedance value and a minimal impedance value;
   scaling an impedance-proximity response profile according to the maximal impedance value and the minimal impedance value to obtain a respective impedance-proximity response curve;
   determining the first threshold value and the second threshold value according to the respective impedance-proximity response curve; and
   defining the respective graphical features index according to the first threshold value and the second threshold value.
iv. wherein the respective graphical features index includes a first visual feature that represents impedance values below a first threshold value corresponding to baseline tissue contact, a second visual feature that represents impedance values above a second threshold value corresponding to contact saturation, and a plurality of other visual features that represent, respectively, a plurality of respective impedance values between the first threshold value and the second threshold value.
v. Wherein the visual feature is color.
vi. Wherein the visual feature is shading in grey scale.
vii. Wherein the visual feature is a rate of flashing.

According to a second aspect of the presently disclosed subject matter there is provided a graphical user interface (GUI) for providing real-time visual feedback of intraluminal catheter engagement using a catheter that comprises one or more electrodes placed on a catheter distal end assembly; the GUI being executable by a computer to:
render on a display a graphical representation of the catheter distal end assembly including one or more graphical elements, each graphical element graphically representing a respective electrode of the one or more electrodes; wherein each graphical element is assigned with a visual feature selected according to a measured impedance value;
for each electrode of the one or more electrodes:
   identify a range of impedance between a first threshold value corresponding to baseline tissue contact and a second threshold value corresponding to contact saturation for each of the one or more electrodes;
   define a graphical feature index that associates impedance values within the defined range with respective visual features of the graphical representation of the one or more electrodes;
   repeatedly receive impedance measurement values measured by the one or more electrodes, and, responsive to a change detected in an impedance value measured by an electrode, update in real-time a respective visual feature of the graphical representation of the electrode based on the impedance measured and the graphical feature index, thereby providing dynamic visual feedback indicative of quality of intraluminal catheter engagement with a tissue wall.

The presently disclosed subject matter further contemplates a computer system comprising at least one processing circuitry configured to provide real-time visual feedback of intraluminal catheter engagement using a catheter that comprises one or more electrodes placed on a catheter distal end assembly as disclosed according to the first aspect above, wherein the system is operatively connectable to a catheter-based electrophysiology mapping system.

The presently disclosed subject matter further contemplates a computer system comprising at least one processing circuitry, configured to execute a method of augmenting intraluminal catheter therapy (ICT) as disclosed according to the first aspect above, wherein the system is operatively connectable to a catheter-based electrophysiology mapping system.

The presently disclosed subject matter further contemplates a non-transitory computer readable storage medium tangibly embodying a program of instructions that, when executed by a computer, cause the computer to perform a method of providing real-time visual feedback of intraluminal catheter engagement using a catheter that comprises one or more electrodes placed on a catheter distal end assembly as disclosed according to the first aspect above.

The presently disclosed subject matter further contemplates a computer program product comprising instructions which, when the program is executed by a computer, cause the computer to carry out a method of augmenting Intraluminal Catheter Therapy (ICT) as disclosed according to the first aspect above.

The GUI, the systems, the computer program product, and the non-transitory program storage device, can optionally comprise one or more of features (i) to (vii) listed above, mutatis mutandis, in any technically possible combination or permutation.

It will also be understood that the system according to the presently disclosed subject matter may be a suitably programmed computer. Likewise, the presently disclosed subject matter contemplates a computer program being readable by a computer for executing the method of the presently disclosed subject matter. The presently disclosed subject matter further contemplates a machine-readable non-transitory memory tangibly embodying a program of instructions executable by the machine for executing the method of the presently disclosed subject matter.

It is to be understood that the presently disclosed subject matter is not limited in its application to the details set forth in the description contained herein or illustrated in the drawings. The presently disclosed subject matter is capable of other embodiments and of being practiced and carried out in various ways. Hence, it is to be understood that the phraseology and terminology employed herein are for the purpose of description and should not be regarded as limiting. As such, those skilled in the art will appreciate that the conception upon which this disclosure is based may readily be utilized as a basis for designing other structures, methods, and systems for carrying out the several purposes of the present presently disclosed subject matter.

## Claims

1. A computer-implemented method of providing real-time visual feedback of intraluminal catheter engagement using a catheter that comprises one or more electrodes placed on a catheter distal end assembly, the method comprising:
while a catheter is in a luminal organ of a patient:
rendering on a display a graphical representation of the catheter distal end assembly and the one or more electrodes thereon;
for each electrode of the one or more electrodes:
identifying a range of impedance between a first threshold value corresponding to baseline tissue contact and a second threshold value corresponding to contact saturation for each of the one or more electrodes;
defining a graphical feature index that associates impedance values within the defined range with respective visual features of the graphical representation of the one or more electrodes;
repeatedly measuring impedance at each of the one or more electrodes; and
dynamically updating a respective visual feature of the graphical representation of each of the one or more electrodes based on the impedance measured and the graphical feature index, thereby providing dynamic visual feedback indicative of quality of intraluminal catheter engagement with a tissue wall.

2. The computer-implemented method of claim 1, wherein the luminal organ is a heart of the patient.

3. The computer-implemented method of claim 1, wherein the distal end assembly is a basket comprising multiple splines, the electrodes being distributed on the splines.

4. The computer-implemented method of claim 1 further comprising determining for each electrode a respective impedance-proximity response curve, comprising:
obtaining by the electrode a plurality of impedance values;
identifying a maximal impedance value and a minimal impedance value;
scaling an impedance-proximity response profile according to the maximal impedance value and the minimal impedance value to obtain a respective impedance-proximity response curve;
determining the first threshold value and the second threshold value according to the respective impedance-proximity response curve; and
defining the respective graphical features index according to first threshold value and the second threshold value.

5. The method of claim 1, wherein the respective graphical features index includes a first visual feature that represents impedance values below a first threshold value corresponding to baseline tissue contact, a second visual feature that represents impedance values above a second threshold value corresponding to contact saturation, and a plurality of other visual features that represent, respectively, a plurality of respective impedance values between the first threshold value and the second threshold value.

6. The computer-implemented method of claim 1, wherein the visual feature is color, or is shading in a gray scale.

7. The computer implemented method of claim 5, wherein the visual feature is a rate of flashing.

8. The computer implemented method of claim 1 comprising rendering on the display a graphical representation of the luminal organ.

9. A graphical user interface (GUI) for providing real-time visual feedback of intraluminal catheter engagement using a catheter that comprises one or more electrodes placed on a catheter distal end assembly, the GUI being executable by a computer to:
render on a display a graphical representation of the catheter distal end assembly including one or more graphical elements, each graphical element graphically representing a respective electrode of the one or more electrodes; wherein each graphical element is assigned with a visual feature selected according to a measured impedance value;
for each electrode of the one or more electrodes:
identify a range of impedance between a first threshold value corresponding to baseline tissue contact and a second threshold value corresponding to contact saturation for each of the one or more electrodes;
define a graphical feature index that associates impedance values within the defined range with respective visual features of the graphical representation of the one or more electrodes;
repeatedly receive impedance measurement values measured by the one or more electrodes, and, responsive to a change detected in an impedance value measured by an electrode, update in real-time a respective visual feature of the graphical representation of the electrode based on the impedance measured and the graphical feature index, thereby providing dynamic visual feedback indicative of quality of intraluminal catheter engagement with a tissue wall.

10. The GUI of claim 9 wherein the visual feature is any one of:
color; shading in grey level; shape; and rate of flashing.

11. A computer system comprising at least one processing circuitry, configured to execute a method of providing real-time visual feedback of intraluminal catheter engagement using a catheter that comprises one or more electrodes placed on a catheter distal end assembly, the method comprising:
while a catheter is in a luminal organ of a patient:
rendering on a display a graphical representation of the catheter distal end assembly and the one or more electrodes thereon;
for each electrode of the one or more electrodes:
identifying a range of impedance between a first threshold value corresponding to baseline tissue contact and a second threshold value corresponding to contact saturation for each of the one or more electrodes;
defining a graphical feature index that associates impedance values within the defined range with respective visual features of the graphical representation of the one or more electrodes;
repeatedly measuring impedance at each of the one or more electrodes; and
dynamically updating a respective visual feature of the graphical representation of each of the one or more electrodes based on the impedance measured and the graphical feature index, thereby providing dynamic visual feedback indicative of quality of intraluminal catheter engagement with a tissue wall.

12. The system of claim 11 is operatively connectable to a catheter-based electrophysiology mapping system comprising the catheter and wherein the luminal organ is a heart of the patient.

13. The system of claim 12 further comprising the catheter, wherein the distal end assembly is a basket comprising multiple splines, the electrodes being distributed on the splines.

14. The system of claim 11 wherein the visual feature is any one of: color; shading in grey level; shape; and rate of flashing.

15. A non-transitory computer readable storage medium tangibly embodying a program of instructions that, when executed by a computer, cause the computer to perform a method of providing real-time visual feedback of intraluminal catheter engagement using a catheter that comprises one or more electrodes placed on a catheter distal end assembly, the method comprising:
while a catheter is in a luminal organ of a patient:
rendering on a display a graphical representation of the catheter distal end assembly and the one or more electrodes thereon;
for each electrode of the one or more electrodes:
identifying a range of impedance between a first threshold value corresponding to baseline tissue contact and a second threshold value corresponding to contact saturation for each of the one or more electrodes;
defining a graphical feature index that associates impedance values within the defined range with respective visual features of the graphical representation of the one or more electrodes;
repeatedly measuring impedance at each of the one or more electrodes; and
dynamically updating a respective visual feature of the graphical representation of each of the one or more electrodes based on the impedance measured and the graphical feature index, thereby providing dynamic visual feedback indicative of quality of intraluminal catheter engagement with a tissue wall.
